(19) Europäisches Patentamt
European Patent Office
Office européen des brevets

(11) Veröffentlichungsnummer: **0 418 674 A1**

# (12) EUROPÄISCHE PATENTANMELDUNG

(21) Anmeldenummer: **90117309.6**

(22) Anmeldetag: **07.09.90**

(51) Int. Cl.[5]: **A61K 31/545**, A61K 31/43, A61K 31/71, A61K 9/02, A61K 47/14, A61K 47/12

(30) Priorität: **11.09.89 US 405494**

(43) Veröffentlichungstag der Anmeldung: **27.03.91 Patentblatt 91/13**

(84) Benannte Vertragsstaaten: **AT BE CH DE DK FR GB IT LI LU NL SE**

(71) Anmelder: **F. HOFFMANN-LA ROCHE AG**
**Postfach 3255**
**CH-4002 Basel(CH)**

(72) Erfinder: **Bachynsky, Maria Oksana**
**38 Carrie Court**
**Nutley, N.J. 07110(US)**
Erfinder: **Davidovich, Alberto**
**29 Kimberly Place**
**Wayne, N.J. 07470(US)**
Erfinder: **Infeld, Martin Howard**
**6 Tuers Place**
**Upper Montclair N.J. 07043(US)**
Erfinder: **Shah, Navnit**
**203 Beverly Hill Road**
**Clifton, N.J. 07012(US)**
Erfinder: **Unowsky, Joel**
**203 East McClellan Avenue**
**Livingston, N.J. 07039(US)**

(74) Vertreter: **Lederer, Franz, Dr. et al**
**Lederer, Keller & Riederer, Patentanwälte,**
**Lucile-Grahn-Strasse 22**
**W-8000 München 80(DE)**

## (54) Pharmazeutische Präparate zur verbesserten Resorption anti-bakterieller Verbindungen.

(57) Pharmazeutische Präparate, enthaltend (a) eine antibakteriell wirksame Verbindung, und (b) eine wirksame Menge eines resorptionsverstärkenden Systems, enthaltend (1) einen Polyoxyäthylenglykol-$C_6$-$C_{18}$-carbonsäure-glyzeridester, und (2) eine $C_6$-$C_{18}$-Carbonsäure gegebenenfalls zusätzlich ein pharmazeutisch anwendbares Salz einer solchen Säure.

EP 0 418 674 A1

# PHARMAZEUTISCHE PRÄPARATE ZUR VERBESSERTEN RESORPTION ANTI-BAKTERIELLER VERBINDUNGEN

Die vorliegende Erfindung betrifft pharmazeutische Präparate, enthaltend (a) eine antibakteriell wirksame Verbindung und (b) eine wirksame Menge eines resorptionsverstärkenden Systems, enthaltend (1) einen Polyoxyäthylenglykol-$C_6$-$C_{18}$-Carbonsäureglyzeridester, und (2) eine $C_6C_{18}$-Carbonsäure, und gegebenenfalls zusätzlich ein Salz einer solchen Säure. Gewünschtenfalls kann das erfindungsgemässe Präparat auch einen pharmazeutisch inerten Träger enthalten.

Der hier verwendete Ausdruck "Polyoxyäthylenglykol-$C_6$-$C_{18}$-Carbonsäureglyzeridester" bezieht sich auf Reaktionsprodukte, die aus der gleichzeitigen Reaktion von Polyoxyäthylenglykol (oder einem polymerisierbaren Vorläufer davon, wie Aethylenoxid) mit einer $C_6$-$C_{18}$-Carbonsäure und Glyzerin oder mit einem $C_6$-$C_{18}$-Carbonsäureglyzerid oder Glyzeriden erhalten wird. Typische Produkte dieser Art sind Gemische von Polyoxyäthylenglykol-$C_6$-$C_{18}$-Carbonsäureglyzeridestern, z.B. PEG-Glyzerincaprat, PEG-Glyzerincaprylat oder PEG-Glyzerincaprylat/Caprat, Polyoxyäthylenglykol-$C_6$-$C_{18}$-Carbonsäureester, z.B. PEG-Caprat, PEG--Caprylat oder PEG-Caprylat/Caprat und Glyzeryl-$C_6$-$C_{18}$- Carbonsäureester, z.B. Glyzerylmono-, di- oder tricaprylat, Glyzerylmono-, di- oder tricaprat oder Glyzerylmono-, di- oder tricaprylat/caprat.

Es wurde gefunden, dass das oben bezeichnete resorptionsverstärkende System zu einer Erhöhung des Ausmasses der Resorption antibakterieller Verbindungen durch das Schleimhautgewebe und in die Blutbahn führt. Die Erfindung fördert somit die Resorption und bewirkt adäquate Blutspiegel antibakterieller Verbindungen, die bei Verabreichung ohne den Resorptionsverstärker auf anderem Wege als parenteral nur schlecht oder überhaupt nicht in nennenswertem Mass resorbiert werden. Damit wird die Herstellung und die Verwendung einer grösseren Anzahl von Dosierungsformen für solche Verbindungen möglich gemacht. Die erfindungsgemässen pharmazeutischen Präparate fördern auch die Resorption und bewirken adäquate Blutspiegel antibakterieller Verbindungen, die sonst durch Schleimhautgewebe nur mässig resorbiert werden und verstärken daher auch die Wirksamkeit solcher therapeutischer Verbindungen.

Die Erfindung betrifft pharmazeutische Kompositionen zur Verabreichung in jeder Dosierungsform, die für die orale oder rektale Verabreichung geeignet ist. Inbegriffen sind dabei orale und rektale Typen pharmazeutischer Präparate, die wirksame Mengen einer antibakteriellen Verbindung und des erfindungsgemässen Resorptionsverstärkungssystems mit oder ohne Anwesenheit inerter Träger und pharmazeutisch anwendbarer Hilfsmittel enthalten.

Die Ausdrücke "antibakteriell" und "antibiotisch" werden hier auswechselbar verwendet und bezeichnen bakterizide oder bakteriostatische Verbindungen, die als Stoffwechselprodukte eines Mikroorganismus, auf synthetischem Wege oder durch eine Kombination mikrobieller und chemischer Verfahren (semi-synthetisch) erhalten wurden.

Für die Anwendung in der vorliegenden Erfindung kommt jede antibiotische Substanz in Betracht, die zur Bekämpfung einer bakteriellen Infektion in einem Wirt geeignet ist, einschliesslich solcher Antibiotika, die nur mässig resorbiert werden, wenn sie nicht infiziert oder nicht infundiert werden. Das wichtigste Gebiet der Erfindung liegt in der Anwendung zur Verstärkung der Resorption und Bioverfügbarkeit von Antibiotika, die grösstenteils nur durch Injektion oder Infusion effektiv verabreicht werden können, weil sie mit anderen Verabreichungswegen nicht oder schlecht resorbiert werden.

Unter den bevorzugten antibakteriellen Verbindungen die als therapeutische Substanz erfindungsgemäss verwendet werden können sind ß-Lactamantibiotika, insbesondere Verbindungen mit einem ß-Lactamring als zentrale Struktur, d.h. mit dem Strukturelement

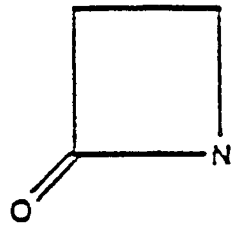

die in verschiedenen Stellungen des Rings substituiert sein können oder die mit anderen Ringen oder Ringsystemen, die für sich wiederum substituiert oder unsubstituiert sein können, kondensiert sein können.

Beispiele solcher ß-Lactamantibiotika sind Penicilline, Cephalosporine, Peneme, Pename, Carbapeneme Carbapename und monocyclische ß-Lactame.

Besonders bevorzugte ß-Lactamantibiotika zur Anwendung der vorliegenden Erfindung sind Verbindungen der Formel

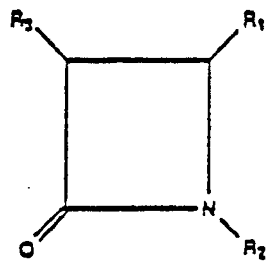

worin $R_1$ wasserstoff, Alkyl oder substituiertes Alkyl, $R_2$ $SO_3$ -M+, M+ ein Proton oder ein Kation, $R_3$ eine Acylaminogruppe oder Hydroxyalkyl oder $R_1$ und $R_2$ zusammen mit dem ß-Lactam (Azetidinon)-Ring, an den sie gebunden sind die Gruppe

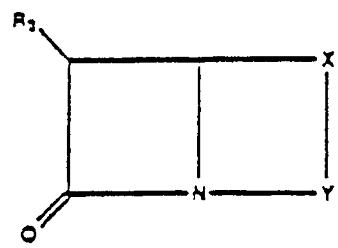

darstellen, worin X -S-, -O, -SO-, $-SO_2$. $-CH_2$ oder $-CH(CH_3)$ ist und Y eine Gruppe

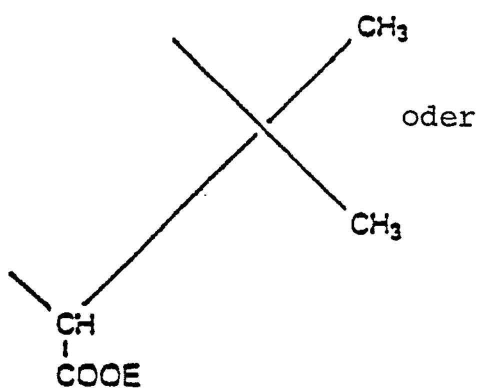

oder

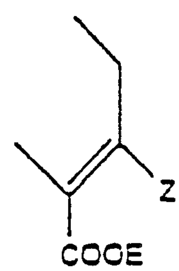

oder

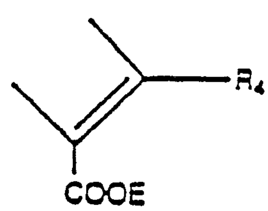

bedeutet, worin $R_4$ eine substituierte Thiogruppe, wie Aethylthio,

$$-SCH_2CH_2NH_2,$$

$$-SCH_2CH_2NH\overset{\overset{NH}{\|}}{C}H, \quad -SCH_2CH_2O\overset{\overset{O}{\|}}{C}NH_2,$$

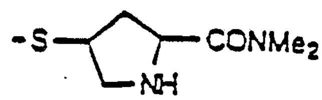

oder eine fakultativ substituierte niedere Alkylgruppe wie Aminomethyl, Acylaminomethyl,

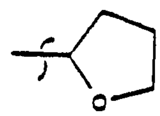

oder eine substituierte Oxygruppe wie Carbamoyloxy

$$(-O\overset{\overset{O}{\|}}{C}NH_2),$$

darstellt, das C-Atom, das die -COOE-Gruppe trägt, an das Stickstoffatom des ß-Lactamrings gebunden ist, Z Wasserstoffe Halogen, Alkoxy oder $CH_2T$ ist, wobei T Wasserstoff, Alkyl, -CO-O-, Pyridinium, Carboxamidopyridinium, Aminopyridinium, Carbamoyloxy, Azido, Cyano, Hydroxyl, die Gruppe -S-Phenyl darstellt, die substituiert sein kann, oder die Gruppe -S-het darstellt, worin "het" ein fakultativ substituierter 5- oder 6-gliedriger heterocyclischer Ring ist und wobei E Wasserstoff, eine pharmazeutisch anwendbarer Estergruppe oder ein salzbildendes Kation darstellt.

Beispiele 5- oder 6-gliedriger heterocyclischer Ringe "het" sind die folgenden:

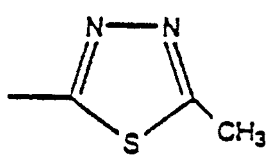

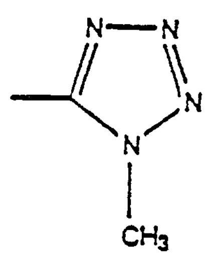

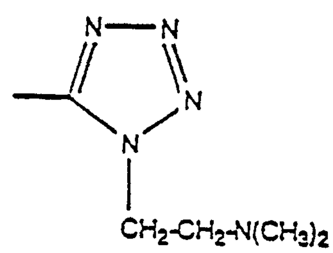

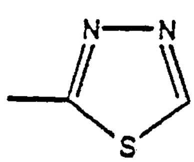

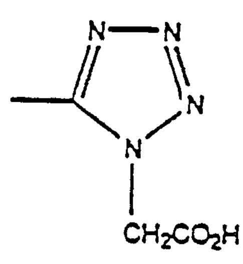

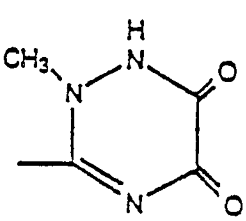

Besonders bevorzugte ß-Lactamantibiotika und deren pharmazeutisch anwendbare Salze, Ester und Hydrate sind Ceftriaxon, ein Cephalosporin, das in der U.S. Patentschrift 4,327,210 beschrieben ist; Carumonam, ein monocyclisches ß-Lactame das in der Europäischen Patentschrift EP 73061 beschrieben ist; Piperacillin, ein Penicillin, das in der U.S. Patentschrift 4,112,090 beschrieben ist; Cefamandol, ein Cephalosporin, das in der U.S. Patentschrift 3,641,021 beschrieben ist; Mezlocillin, ein Penicillin, das in der U.S. Patentschrift 3,974,142 beschrieben ist: und Cefazolin. ein Cephalosporin, das in der U.S. Patentschrift 3,516,997 beschrieben ist. Weitere Beispiele solcher Verbindungen sind Cefoxithin, Cefmetazol, Cefotetan, Moxalactam, Cefuroxim, Ceforamid, Cefoperazon, Ceftizoxim, Cefotaxim, Cefmenoxim, Ceftazidim, Cefsulodin, Cefazolin, Cephalexin, Azlocillin, Penicillin G, Temocillin, Sulbenicillin, Ticarcillin, Mecillinam, Amoxicillin, Methicillin, Carbenicillin, Thienamycin, N-Formimidoylthienomycin, Sulbactam und Azthreonam.

Weiterhin umfasst die Erfindung die Anwendung auf Antibiotika, die keine ß-Lactame sind, beispielsweise Vancomycin und Gentamicin, deren Resorption und Bioverfügbarkeit durch die Anwendung des erfindungsgemässen Resorptionsverstärkungssystems verbessert werden.

Die Komponente (b)(1) des erfindungsgemässen resorptionsverstärkenden Systems kann das Produkt einer Veresterung zwischen einem Polyäthylenglykol, Glyzerin und einem oder mehreren geradkettig oder verzweigtkettigen $C_6$-$C_{18}$-Carbonsäuren seine vorzugsweise einer monofunktionalen Säure oder Säuren. Alternativ kann die Komponente (b)(1) durch Oligomerisierung oder Polymerisierung von Aethylenoxid in Gegenwart eines Glyzerinesters und einer oder mehrerer $C_6$-$C_{18}$-Carbonsäuren erhalten werden (Glyzeridester). Eine weitere bevorzugte Möglichkeit ist die gleichzeitige Reaktion des Glyzeridesters oder der Glyzeridester mit einem völlig vorgeformten Polyoxyäthylenglykol unter Bedingungen die geeignet sind, Alkoholyse zu erreichen.

Nach einem bevorzugten Verfahren, das unter Alkoholyse verläuft, wird ein Reaktionsgefäss mit stöchiometrischen Quantitäten eines Glyzerinfettsäureesters oder Fettsäureestern und einem Polyäthylenglykol beschickt. Das Reaktionsgefäss wird verschlossen und unter Atmosphärendruck auf 200°C erwarmt, wobei bei 70°C mit Rühren begonnen wird und das Rühren für eine Dauer von 12-24 Stunden oder bis zur Beendigung der Reaktion fortgesetzt wird. Das Reaktionsgefäss wird dann abkühlen gelassen und das Reaktionsprodukt aus dem Reaktionsgemisch abfiltriert.

Beispiele von gesättigten oder von ungesättigten $C_6$-$C_{18}$-Carbonsäuren, die für die Komponente (b)(1) des Resorptionsverstärkungssystems Verwendung finden können, sind Capronsäure, Caprylsäure, Caprinsäure, Laurinsäure, Myristinsäure, Oelsäure, Palmitin- und Stearinsäure. Besonders bevorzugt sind Caprin- und Caprylsäure, einzeln oder zusammen.

Das Polyoxyäthylenglykol, das bei der Bildung der Komponente (b)(1) angewandt wird, ist typischerweise ein solches von mittlerem bis hohem Molekulargewicht, vorzugsweise mit einem Molekulargewicht im Bereich von 200-1500, vorzugsweise 300-600.

Ein Material, das als Komponente (b)(1) besonders geeignet ist, hat die folgenden Charakteristiken:
Organoleptische Eigenschaften:
Aussehen: klar ölige Flüssigkeit
Geruch: schwach
Farbe: hellgelb bis gelb
Physikalische und chemische Eigenschaften:
Säurewert: 0,2-0,6
Sulfatierte Asche: weniger als 0,05%
Verseifungsindex: 85-105
Jodindex: weniger als 2
Feuchtigkeitsgehalt: weniger als 0,05%
Gehalt an freiem Glyzerin: etwa 2%
Gehalt an Monoglyzeriden: etwa 6-8%
Dichte $[d]_{20}^{4}$ ): 1,062-1,068 g/ml
Brechungsindex $[n]_{D}^{20}$ ): 1,458-1,462

Eine für das erfindungsgemässe Resorptionsverstärkungssystem geeignete Komponente ist unter dem Namen LABRASOL (PEG-8-Caprylat/Capratglyzeridester) Gattefosse Corporation, Paris und SOFTIGEN 767 (PEG-6-Caprylat/Capratglyzeridester) Dynamit Nobel, BRD, im Handel.

Die als Komponente (b)(2) des erfindungsgemässen Resorptionsverstärkungssystems verwendete $C_6$-$C_{18}$-Carbonsäure oder deren Salze leitet sich von einer acyclischen Carbonsäure ab, die geradkettig oder verzweigt sein kann. Beispiele sind Capronsäure, Caprylsäure, Caprinsäure, Laurinsäure, Myristinsäure, Oelsäure, Palmitinsäure und Stearinsäure. Besonders bevorzugt ist Caprylsäure..

$C_6$-$C_{18}$-Carbonsäuresalze können in herkömmlicher Weise hergestellt werden, durch Umsetzung der Säure mit einer Base, die ein nicht toxisches pharmakologisches, pharmazeutisches annehmbares Kation

hat. Im allgemeinen ist jede Base geeignet, die ein Salz mit einer Carbonsäure bildet und deren pharmakologische Eigenschaften keine abträglichen physiologischen Eigenschaften besitzen, wenn sie eingenommen oder auf andere Weise einem Warmblüter verabreicht werden. Solche Basen sind beispielsweise Alkalimetall- und Erdalkalimetallhydroxide oder -carbonate wie Natriumhydroxid, Kaliumhydroxid, Calciumhydroxid, Kaliumcarbonat und ähnliches. Besonders bevorzugt sind Natriumsalze, hauptsächlich wegen ihrer leichten Zugänglichkeit. Natriumcaprylat ist besonders bevorzugt.

Das $C_6$-$C_{18}$-Carbonsäuresalz ist als Stabilisierungsmittel für die $C_6$-$C_{18}$-Carbonsäure von Wert und ist besonders erwünscht für Präparate, in denen das Antibiotikum Ceftraxon ist.

Die relativen Mengenverhältnisse der beiden Komponenten, die im erfindungsgemässen Resorptionsverstärkungssystem enthalten sind, können variiert werden, um optimale Resultate für eine bestimmte Ausführungsform der Erfindung zu erreichen. Vorzugsweise ist die Komponente (b) (1) in Mengen von etwa 20 bis etwa 80% und die Carbonsäure in Mengen von etwa 80 bis etwa 20% bezogen auf das Gesamtgewicht (100%) der beiden Komponenten anwesend. Besonders bevorzugt sind Mengenverhältnisse von etwa 40 bis etwa 60% der Komponente (b) (1) und etwa 60 bis etwa 40% der Carbonsäure, insbesondere jeweils 50%, bezogen auf das Gewicht beider Komponenten zusammen. Für ein 3-Komponenten-System ist die Komponente (b)(1) vorzugsweise in Mengen von etwa 20 bis etwa 80%, insbesondere etwa 40 bis etwa 60% und äusserst bevorzugt in Mengen von etwa 50% anwesend, bezogen auf das Gesamtgewicht der drei Komponenten. Die Carbonsäure ist vorzugsweise in Mengen von etwa 10 bis etwa 40%, insbesondere von etwa 15 bis etwa 25% und äusserst bevorzugt in Mengen von etwa 25% bezogen auf das Gesamtgewicht anwesend. Das Carbonsäuresalz ist vorzugsweise in Mengen von etwa 10 bis etwa 40%, insbesondere von etwa 25 bis etwa 35% und äusserst bevorzugt von etwa 25% bezogen auf das Gesamtgewicht anwesend.

Die wirksame Menge der Komponente (b) in dem erfindungsgemässen Präparat hängt von Faktoren wie der besonderen antibakteriellen Verbindung die angewandt wird und deren Menge ab, wie auch vom Alter des zu behandelnden Patienten.

Im allgemeinen ist es für orale Dosierungsformen bevorzugt, etwa 50 bis 1000 mg, insbesondere 100 bis etwa 500 mg des Resorptionsverstärkungssystems pro Dosiseinheit des pharmazeutischen Präparates anzuwenden. Diese Präparate enthalten üblicherweise die antibakterielle Verbindung in Mengen von etwa 10 bis etwa 500 mg, insbesondere von etwa 50 bis etwa 250 mg pro Dosiseinheit.

Rektale Dosierungsformen enthalten üblicherweise etwa 25 bis etwa 1500 mg, insbesondere 100 bis etwa 500 mg des Resorptionsverstärkungssystems pro Dosiseinheit. Solche Präparate enthalten die antibakterielle Verbindung üblicherweise in Mengen von etwa 10 bis etwa 3000 mg, insbesondere von etwa 100 bis etwa 1500 mg pro Dosiseinheit.

Der Ausdruck "Dosiseinheit" wird hier im üblichen Sinne gebraucht, um eine einzelne Darreichung des Medikamentes in der angegebenen Menge zu bezeichnen. Diese Menge kann in Form einer einzelnen Pille, Tablette, Kapsel oder Suppositorium verabreicht werden oder alternativ in Vielfachen von zwei oder mehr solcher Dosiseinheiten, die dann zusammen die angegebene Menge des Wirkstoffs enthalten.

Die beschriebene antibakterielle Verbindung und das Resorptionsverstärkungssystem, können in einen Träger eingearbeitet werden, falls dies gewünscht ist. Als Träger kann jeder pharmazeutisch anwendbare Feststoff, Halbfeststoff oder flüssige Träger, in dem diese Komponenten löslich oder leicht verteilbar sind, angewandt werden. Beispiele dafür sind Kakaobutter, Polyäthylenglykole, Polypropylenglykole, Methylcellulose, Carboxymethylcellulose und semi-synthetische Basen, wie Suppocire® (Gattefosse Corp., Paris). Vorzugsweise ist das Vehikel ein Feststoff. Bevorzugt als Feststoff für die erfindungsgemässe Anwendung sind Gemische von Mono-, Di- und Triglyzeriden von $C_{12}$-$C_{18}$ natürlichen gesättigten Fettsäuren, vorzugsweise Fettsäuren, die eine gerade Anzahl von C-Atomen haben ($C_{12}$, $C_{14}$, $C_{16}$). Besonders geeignet und bevorzugt sind pharmazeutische Basen von Dynamit Nobel unter der Handelsbezeichnung "WITEPSOL".

Andere pharmazeutisch verträgliche Trägermaterialien können gewünschtenfalls je nach den besonderen Bedürfnissen angewandt werden. Ihre Auswahl liegt im Bereich des Fachwissens.

Falls ein Trägerstoff angewandt wird, liegen seine Mengen in Bereichen, die für pharmazeutische Trägermaterialien üblicherweise angewandt werden und die sicher verabreicht werden können.

Für die orale Verabreichung der erfindungsgemäsen Präparate ist eine magensaftresistent überzogene Formulierung bevorzugt, vorzugsweise eine solche in fester Form. Die Formulierung kann in Hartgelatine- oder Weichgelatinekapseln oder, falls sie flüssig ist, auf einem geeigneten Träger absorbiert sein unter Bildung eines freifliessenen Pulvers und dann in die Kapsel abgefüllt oder alternativ zu Pillen oder Tabletten verpresst sein. Andere Dosierungsformen sind Mikrokapseln oder Beadlets, bestehend aus der antibakteriellen Verbindung und dem Resorptionsverstärkungssystem, die in eine mit einem magensaftresistenten Ueberzug versehenen Kapsel eingehüllt sind.

Die Verwendung von magensaftresistenten Ueberzügen dient dazu, die antibakterielle Verbindung vor

der Magenflüssigkeit zu schützen und einen optimalen Transport der antibakteriellen Verbindung zusammen mit dem Resorptionsverstärkungssystem in den Darmtrakt zu erreichen. Der magensaftresistente Ueberzug ist zum grössten Teil gegenüber der Magenflüssigkeit widerstandsfähig und bleibt unangegriffen, löst sich aber in der Darmflüssigkeit, wobei der Wirkstoff freigesetzt wird.

Die Wirksamkeit eines besonderen magensaftresistenten Ueberzugsmaterials kann mit bekannten Verfahren gemessen werden. Beispielsweise sind geeignete magensaftresistente Ueberzugsmaterialien zur Anwendung in der vorliegenden Erfindung die folgenden:

Celluloseacetatphthalat
Celluloseacetattrimellitat
Hydroxypropylmethylcellulosephthalat
Hydroxypropylmethylcellulosephthalatsuccinat
Polyvinylacetatphthalat
Methacrylsäure
Methacrylsäureester

Diese Materialien können mit oder ohne Weichmacher, wie acetylierten Glyzeriden oder Diäthylphthalat in an sich bekannter Weise angewandt werden.

Der Prozentsatz des angewandten Ueberzuges liegt üblicherweise zwischen etwa 1 und etwa 10 Gewichtsprozenten oder mehr, insbesondere zwischen etwa 2 bis etwa 8 Gewichtsprozent bezogen auf das Gesamtgewicht der Dosiseinheitsform., d.h. der gesamten Kapsel oder dem gesamten Tablettengewicht. Beispiele geeigneter Ueberzugsformulierungen sind nachstehend angegeben.

## Formulierungen für magensaftresistente Ueberzüge

| Inhaltsstoff | Gewichtsprozent |
|---|---|
| **Präparat A:** | |
| Hydroxypropylmethylcellulosephthalat (HPMCP) | 5.0 |
| Triacetin | 0.5 |
| Methylenchlorid | 47.25 |
| Denaturierter Alkohol | 47.25 |
| **Präparat B:** | |
| HPMCP | 10.0 |
| Titandioxid | 0.2 |
| Triäthylcitrat | 1.5 |
| Aceton | 44.15 |
| Denaturierter Alkohol | 44.15 |
| **Präparat C:** | |
| Celluloseacetatphthalat (CAP) | 8.5 |
| Diäthylphthalat | 1.5 |
| Titandioxid | 0.2 |
| Aceton | 44.9 |
| Denaturierter Alkohol | 44.9 |

Präparat D:

| | |
|---|---|
| Polyvinylacetatphthalat | 5.0 |
| Acetylierte Glyzeride | 0.8 |
| Methylenchlorid | 47.1 |
| Denaturierter Alkohol | 47.1 |

Präparat E:

| | |
|---|---|
| Methacrylsäure oder Methacrylsäure-ester (Eudragit S or L, Rohm Pharma, GMBH, Wetterstadt, BRD) | 8.0 |
| Aceton | 46.0 |
| Absoluter Alkohol | 46.0 |
| Triacetin | 1.0 |

Die erfindungsgemäsen oralen Formulierungen können auch zusätzlich übliche Zusätze oder Hilfsstoffe enthalten in für solche Materialien üblichen Mengen. Beispielsweise können solche Zusätze Verdickungsmittel sein, wie Kieselsäure (beispielsweise Aerosilprodukte) Bentonite, colloidaler Ton, Carboxymethylcellulose, modifizierte Montmorillonite wie Alkylammoniumsalze von Montmorilloniten (beispielsweise Handelsprodukte wie Bentone) organische Verdickungsmittel und Strukturbildner wie gesättigte höhere Fettsäuren und Alkohole mit 12-20 C-Atomen (beispielsweise Stearin- und Palmitinsäure oder Stearin und Cetylalkohol) Wachse, Monoglyzeride, gesättigter oder ungesättigter höherer Fettsäuren, wie Stearinsäure, Palmitinsäure oder Oelsäure, Geliermittel wie Aluminiumstearat, Dispergierungsmittel, wie ionische. nicht-ionische oder kationische oberflächenaktive Stoffe, Emulgatoren, wie Lezithin usw.

Die erfindungsgemässen Kompositionen können auch pharmazeutisch anwendbare Hilfsmittel wie Bindemittel oder Schmiermittel zum Tablettieren, Stabilisatoren, Antioxidan tien, Fliessmittel (zur Verbesserung der Giessfähigkeit oder Fliessfähigkeit während der Verarbeitung), Konservierungsmittel, Geschmacksstoffe, Farbstoffe und Puffer enthalten. Alle diese Stoffe können aus Materialien ausgewählt werden, die für solche Anwendungszwecke bekannt sind.

Die verbesserte Resorption von Antibiotika durch Schleimhautgewebe mittels der erfindungsgemäsen Präparate wurde in in vivo-Tests geprüft.

Enterale Resorption in vivo (Ratten)

Weibliche Ratten im Gewicht von etwa 250 g wurden über Nacht fasten gelassen und mit Metofan anästhesiert. Die Antibiotika wurden in Lösung mit oder ohne Resorptionsverstärker nach Bauchschnitt in das Duodenum unterhalb des Magenausgangs injiziert. Für Vergleichszwecke wurde die Lösung alternativ intravenös in die Schwanzvene appliziert. Die Antibiotika wurden in Wasser gelöst, so dass die Lösungen (mit oder ohne Verstärker) 20 mg/kg, etwa 5 mg pro Ratte, enthielten.

Plasmaspiegel des Antibiotikums bei Ratten

Die Konzentration des Antibiotikums im Plasma der Ratte wurde nach verschiedenen Zeitintervallen nach intravenöser oder enteraler Verabreichung bestimmt. Blutproben wurden aus der Schwanzvene des Versuchstiers vor der Verabreichung des Antibiotikums und 5, 10, 20, 40, 60, 120, 240 und 360 Minuten danach entnommen, danach zentrifugiert und das Plasma bis zur Analyse eingefroren.

Bioassay der Plasmaproben

Die meisten der getesteten Antibiotika zeigten einen gewissen Grad von Proteinbindung, wenn das Antibiotikahaltige Plasma gegen das Antibiotikum in Wasser getestet wurde. Antibiotika, die nicht an Plasma gebunden waren, wurden mit Wasser verdünnt und gegen in Wasser hergestellte Standards geprüft. Für gebundene Antibiotika wurde der Einfluss der Proteinbindung eliminiert durch Verdünnen aller Standardlösungen und Proben im gesammelten Plasma. Im Fall von Ceftriaxon und Cefazolin wurde der Effekt der Bindung dadurch berücksichtigt, dass Plasmaproben mit Acetonitril mit einem Verdünnungsfaktor 1:12 deproteinisiert und gegen eine Standardkurve in Wasser verdünnt gemessen wurden. Antibiotikaspiegel wurden wie in Tabelle 1 angegeben auf Agarplatten ermittelt.

Tabelle 1

| Antibiotikum | Testorganismus | Bereich der Standardkurven (μg/ml) | Testmedium | Volumen (μl) |
|---|---|---|---|---|
| Carumonam | E. coli. 1346 | 32-1 | AA#1[2] | 20 |
| Ampicillin[1] | M. lutea ATCC 9341 | 8-0.25 | AA#1 | 20 |
| Cefamandol [1] | M. lutea ATCC 9341 | 32-1 | AA#1 | 20 |
| Cefotaxim [1] | E. coli. 1346 | 8-0.25 oder 16-0.5 | AA#1 | 20 |
| Cefoxitin[1] | S. aureus MB2786 | 64-4 | BHI[3] | 20 |
| Ceftriaxon [1] | E. coli. 1346 | 4-0.125 | AA#1 | 20 |
| Cefazolin[1] | S. aureus ATCC 25923 | 32-1 | AA#1 | 50 |
| | B. subtilis spores | 32-2 | AA#1 | 50 |
| Moxalactam[1] | E. coli. 1346 | 50-1.56 | AA#1 | 50 |
| Penicillin G[1] | M. lutea ATCC 9341 | 8-0.5 | AA#1 | 20 |
| Vancomycin[1] | B. cereus ATCC 11778 | 64-2 | AA#8[5] | 50 |

[1] Antibiotika mit Proteinbindung im Rattenplasma.
[2] Antibiotischer Agar Nr. 1 (Difco).
[3] BHI = Brain Heart Infusion Media (Difco).
[5] Antibiotischer Agar Nr. 8 (Difco).

Die Platten wurden bei 37°C über Nacht bebrütet und die Hemmzonen auf die nächsten 0,1 ml abgelesen. Die Berechnungen wurden mittels einer Autoassaymaschine (Giles Scientific, Inc., New York) vorgenommen. Vergleiche J.V. Bennett et al., Applied Microbiology 14 , 170-177 (1966).

Die Resultate waren wie folgt:

Tabelle 2

| Enterale Resorption bei Ratten mit und ohne Resorptionsverstärker | | | |
|---|---|---|---|
| Dosis = 5 mg/0,5 ml | | | |
| | Cmax (µg/ml) | | |
| Antibiotikum | (Kontrolle) Wasser | Labrasol + Caprylsäure* | Labrasol* + Caprylsäure + Natriumcaprylat** |
| Carumonam | 0 | 13.6 ± 2.9 | 10.9 ± 1.4 |
| Cefamandol | 1.3 ± 2.5 | 5.4 ± 1.5 | 15.5 ± 6.8 |
| Cefazolin | 0 | - | 17.5 ± 5.7 |
| Cefoxitin | 0 | - | 12.8 ± 1.7 |
| Cefotetan | 0 | 17.8 ± 4.9 | 20.4 ± 4.9 |
| Moxalactam | 0 | - | 15.8 ± 1.3 |
| Penicillin G | 0.5 ± 0.1 | 7.2 ± 0.7 | 2.9 ± 1.6 |
| Vancomycin | 2.9 ± 0.6 | 8.0 ± 2.3 | 5.3 ± 2.1 |
| Ceftriaxon | 2.4 ± 1.9 | 57.0 ± 10.2 | 52.2 ± 22.5 |

* Gew.Verhältnis Labrasol:Caprylsäure 2:1

** Gew.Verhältnis Labrasol:Caprylsäure:Natriumcaprylat 4:1:1

Orale Resorption in vivo (Affen)

Erwachsene Affen (Papio anubis und Papio hamadryas) im Gewicht von 12-30 kg wurden über Nacht fasten gelassen, dann durch i.m.-Injektion von Ketaminhydrochlorid vor der Verabreichung des Antibiotikums sediert. Jeder Affe erhielt vier Hartgelatinekapseln durch Schlundsonde mit der folgenden Zusammensetzung:

| | A | B |
|---|---|---|
| Ceftriaxon-Natrium | 315 mg | 315 mg |
| Labrasol | 225 mg | 225 mg |
| Caprylsäure | 200 mg | 100 mg |
| Natriumcaprylat | --- | 100 mg |
| Witepsol H15 | 210 mg | 210 mg |
| | 950 mg | 950 mg |

Die Kapseln waren mit einem Ueberzug von Polyvinylacetatphthalat (etwa 8% des Kapselgewichtes) versehen.

Die Plasmakonzentration von Ceftriaxon wurde vor der Verabreichung und 10, 20, 40, 60, 120, 180 und 240 Minuten danach bestimmt.

Als Resultat wurden 27,7 ± 12,7% Bioverfügbarkeit und 10-39,5 µg/ml $C_{max}$-Bereich für Formulierung A; 31,0 ± 19,8% Bioverfügbarkeit und 34,6 ± 23,2 µg/ml $C_{max}$-Bereich für Formulierung B; und 0% Bioverfügbarkeit und 0 µg/ml $C_{max}$-Bereich für den Kontrollwert (300 mg Ceftriaxon-Na in der gleichen Kapsel ohne Resorptionsverstärker) ermittelt.

Rectale Resorption in vivo (Affen)

Für diesen Versuch wurden männliche und weibliche erwachsene Affen (Papio anubis und Papio hamadryas) im Gewicht vom 12-27 kg verwendet. die über Nacht vor der Verabreichung des Antibiotikums fasten gelassen wurden, dann durch i.m.-Injektionen von Ketamin-Hydrochlorid vor der Verabreichung des

Antibiotikums sediert wurden. Aus den nachstehend beschriebenen Formulierungen wurden Suppositorien hergestellt die den Tieren verabreicht wurden. Die Rektalöffnungen wurden dann mit Klebeband verschlossen, um ein Herausdrücken der Suppositorienmasse zu vermeiden.

| | C | D |
|---|---|---|
| Antibiotikum (aktive Form) | 500 mg | 500 mg |
| Labrasol | 250 mg | 250 mg |
| Caprylsäure | 200 mg | 100 mg |
| Natriumcaprylat | --- | 100 mg |
| Witepsol H15 | 1050 mg | 1050 mg |
| | 2000 mg | 2000 mg |

Zur Messung der Resorption des Antibiotikums in den Blutstrom wurden Blutproben aus der Femoralregion vor der Verabreichung des Antibiotikums und 15, 30, 60, 120, 240, 360 und 480 Minuten danach entnommen. Proben wurden zentrifugiert und wie oben beschrieben vermessen. Die Resultate sind in Tabelle 3 zusammengestellt:

Tabelle 3

| Bioverfügbarkeit und Cmax-Bereich des Antibiotikums in Affen nach rektaler Verabreichung | | | | |
|---|---|---|---|---|
| | Mit Resorptionsverstärker | | Kontrolle ohne Resorptionsverstärker Enhancer System | |
| Antibiotikum | % Bioverfügbarkeit | Cmax Bereich mcg/ml | % Bioverfügbarkeit | Cmax Bereich mcg/ml |
| Formulierung C : | | | | |
| Ceftriaxon | 17.3 ± 4.8 | 21.6-46.6 | 4.3 ± 2.8 | 0.3- 9.0 |
| Cefotetan | 11.4 ± 7.4 | 4.3-21.1 | 4.9 ± 3.1 | 2.4-10.1 |
| Formulierung D : | | | | |
| Ceftriaxon | 17.1 ± 5.0 | 16.3-38.3 | 4.3 ± 2.8 | 0.3- 9.0 |
| Cefotetran | 24.1 ± 12.8 | 2.5-61.5 | 4.9 ± 3.1 | 3.4-10.1 |

Nachstehend sind einige Formulierungen für verschiedene Dosierungsbereiche für die erfindungsgemässen Präparate angeführt. Obschon in diesen Beispielen Ceftriaxon, das bevorzugte Antibiotikum im Rahmen der Erfindung, figuriert, können auch andere Antibiotika in passenden Mengen eingesetzt werden.

| Beispiel 1 | | | | |
|---|---|---|---|---|
| Orale Formulierung | | | | |
| (Hartgelatinekapsel) | | | | |
| | mg/Kapsel | | | |
| Ceftriaxon-Natrium | 120 mg | 300 mg | 120 mg | 300 mg |
| Labrasol | 90 mg | 225 mg | 90 mg | 225 mg |
| Natriumcaprylat | -- | -- | 40 mg | 100 mg |
| Caprylsäure | 80 mg | 200 mg | 40 mg | 100 mg |
| Witepsol H15 | 84 mg | 210 mg | 84 mg | 210 mg |
| | 374 mg | 935 mg | 374 mg | 935 mg |

Herstellung :

Die Base (Witepsol H15) wird auf 55° C erwärmt und die Komponenten des Resorptionsverstärkersystems der Schmelze zugemischt. Die Schmelze wird dann auf 45° C gekühlt und der Wirkstoff (Ceftriaxonnatrium) der geschmolzenen Masse, zugesetzt und gleichmässig darin verteilt. Die Masse wird homogenisiert bis eine einheitliche Suspension erhalten wird. Diese wird dann in Gelatinekapseln abgefüllt, gegebenenfalls versiegelt und die Kapseln mit einem magensaftresistenten Ueberzug (PVAP) versehen.

| Beispiel 2 | | | | |
|---|---|---|---|---|
| Orale Formulierung | | | | |
| (Weichgelatinekapsel) | | | | |
| | mg/Kapsel | | | |
| Ceftriaxon-Natrium | 120 mg | 300 mg | 120 mg | 300 mg |
| Labrasol | 90 mg | 225 mg | 90 mg | 225 mg |
| Natriumcaprylat | -- | -- | 40 mg | 100 mg |
| Caprylsäure | 80 mg | 200 mg | 40 mg | 100 mg |
| Witepsol H15 | 84 mg | 210 mg | 84 mg | 210 mg |
| | 374 mg | 935 mg | 374 mg | 935 mg |

Herstellung :

Die Base (Witepsol H15) wird auf 55° C erwärmt und die Komponenten des Resorptionsverstärkersystems der Schmelze zugemischt. Die Schmelze wird dann auf 45° C gekühlt und der Wirkstoff (Ceftriaxonnatrium) der geschmolzenen Masse, zugesetzt und gleichmässig darin verteilt. Die Masse wird homogenisiert bis eine einheitliche Suspension erhalten wird. Diese wird dann in Weichgelatinekapseln abgefüllt, und mit einem magensaftresistenten Ueberzug wie in Beispiel 1 versehen.

## Beispiel 3

### Orale Formulierung
(Mikrokapsel)

| | mg/Dosis | | | |
|---|---|---|---|---|
| Ceftriaxon-Natrium | 150 mg | 300 mg | 150 mg | 300 mg |
| Labrasol | 90 mg | 180 mg | 90 mg | 180 mg |
| Natriumcaprylat | -- | -- | 40 mg | 80 mg |
| Caprylsäure | 80 mg | 160 mg | 40 mg | 80 mg |
| Witepsol H15 | 150 mg | 300 mg | 150 mg | 300 mg |
| | 470 mg | 940 mg | 470 mg | 940 mg |

Herstellung :

Die Base (Witepsol H15) wird auf 55° C erwärmt und die Komponenten des Resorptionsverstärkersystems der Schmelze zugemischt. Die Schmelze wird dann auf 45° C gekühlt und der Wirkstoff (Ceftriaxonnatrium) der geschmolzenen Masse, zugesetzt und gleichmässig darin verteilt. Die Masse wird homogenisiert bis eine einheitliche Suspension erhalten wird. Die Suspension wird durch Versprühen in Mikrokapseln übereführt, diese in Kapseln abgefüllt und letztere wie in Beispiel 1 magensaftresistent überzogen.

| Beispiel 4<br>Rektale Formulierung<br>(Suppositorium) | | | | | | |
|---|---|---|---|---|---|---|
| | mg/Suppositorium | | | | | |
| Ceftriaxon-Natrium | 120 mg | 300 mg | 600 mg | 120 mg | 300 mg | 600 mg |
| Labrasol | 250 mg | 250 mg | 250 mg | 250 mg | 250 mg | 250 mg |
| Natriumcaprylat | -- | -- | -- | 100 mg | 100 mg | 100 mg |
| Caprylsäure | 200 mg | 200 mg | 200 mg | 100 mg | 100 mg | 100 mg |
| Witepsol H15 | 1430 mg | 1250 mg | 950 mg | 1430 mg | 1250 mg | 950 mg |
| | 2000 mg | 2000 mg | 2000 mg | 2000 mg | 2000 mg | 2000 mg |

Die Base (Witepsol H15) wird auf 55° C erwarmt und die Komponenten des Resorptionsverstärkungssystems der Schmelze zugemischt. Die Schmelze wird dann auf 45° C gekühlt und der Wirkstoff (Ceftriaxonnatrium) der geschmolzenen Masse zugesetzt und gleichmässig darin verteilt. Die Masse wird homogenisiert bis eine einheitliche Suspension erhalten wird, dann in Suppositorienschalen gefüllt und aushärten gelassen.

## Ansprüche

1. Pharmazeutisches Präparat, enthaltend (a) eine antibakteriell wirksame Verbindung, und (b) eine wirksame Menge eines resorptionsverstärkenden Systems, enthaltend (1) einen Polyoxyäthylenglykol-$C_6$-$C_{18}$-

carbonsäureglyzeridester, und (2) eine $C_6$-$C_{18}$-Carbonsäure und gegebenenfalls zusätzlich ein pharmazeutisch anwendbares Salz einer solchen Säure, und gewünschtenfalls einen pharmazeutisch inerten Träger.

2. Präparat nach Anspruch 1 als orale Dosierungsform.

3. Präparat nach Anspruch 2 als magensaftresistent überzogene Dosierungsform.

4. Präparat nach Anspruch 3, wobei die Komponente (a) in Mengen von etwa 10 bis etwa 500 mg und die Komponente (b) in Mengen von etwa 50 bis etwa 1000 mg pro Dosierungseinheit vorliegt.

5. Präparat nach Anspruch 1 als rektale Dosierungsform.

6. Präparat nach Anspruch 5, wobei die Komponente (a) in Mengen von etwa 10 bis etwa 3000 mg und die Komponente (b) in Mengen von etwa 25 bis etwa 1500 mg pro Dosierungseinheit vorliegt.

7. Präparat nach den Ansprüchen 1-6, wobei die Komponente (b) aus etwa 20 bis etwa 80 Gewichts-% Glyceridester und etwa 80 bis etwa 20 Gewichts-% Carbonsäure besteht.

8. Präparat nach den Ansprüchen 1-7, wobei das Polyäthylenglykol der Komponente (b)(1) ein mittleres Molekulargewicht von etwa 200 bis etwa 1500 hat.

9. Präparat nach den Ansprüchen 1-8, wobei die Komponente (b)(1) ein PEG-8-caprylat/caprat-glycerideester; die $C_6$-$C_{18}$-Carbonsäure Caprylsäure und das $C_6$-$C_{18}$-Carbonsäuresalz Natriumcaprylat ist.

10. Präparat nach den Ansprüchen 1-8, worin die antibakteriell wirksame Verbindung ein ß-Lactam der Formel

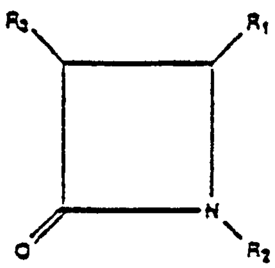

ist,

worin $R_1$ Wasserstoff, Alkyl oder substituiertes Alkyl, $R_2$ $SO_3$-M+, M+ ein Proton oder ein Kation, $R_3$ eine Acylaminogruppe oder Hydroxyalkyl oder $R_1$ und $R_2$ zusammen mit dem ß-Lactam (Azetidinon)-Ring, an den sie gebunden sind die Gruppe

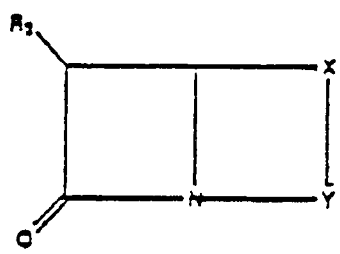

darstellen, worin X -S-, -O, -SO-, -$SO_2$, -$CH_2$ oder -CH($CH_3$) ist und Y eine Gruppe

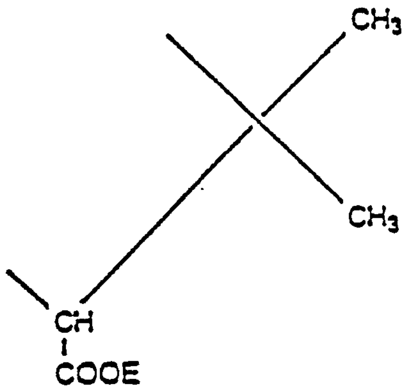

oder

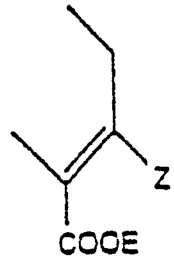

oder

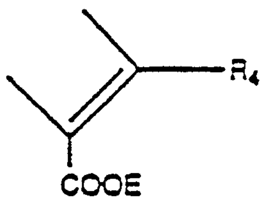

bedeutet, worin $R_4$ Aethylthio, oder eine Gruppe

$-SCH_2CH_2NH_2$,

$-SCH_2CH_2NH\overset{\overset{NH}{\|}}{C}H$, $-SCH_2CH_2O\overset{\overset{O}{\|}}{C}NH_2$,

oder

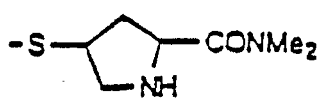

oder Aminomethyl, Acylaminomethyl,

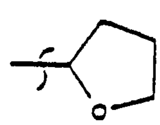

oder Carbamoyloxy

$(-O\overset{\overset{O}{\|}}{C}NH_2)$,

darstellt, das C-Atom, das die -COOE-Gruppe trägt, an das Stickstoffatom des ß-Lactamrings gebunden ist, Z Wasserstoff, Halogen, Alkoxy oder $CH_2T$ ist, wobei T Wasserstoff, Alkyl, -CO-O-, Pyridinium, Carboxamidopyridinium, Aminopyridinium, Carbamoyloxy, Azido, Cyano, Hydroxyl, die Gruppe -S-Phenyl darstellt, die substituiert sein kann, oder die Gruppe -S-het darstellt, worin "het" ein fakultativ substituierter 5- oder 6-gliedriger heterocyclischer Ring ist und wobei E Wasserstoff, ein pharmazeutisch anwendbarer Estergruppe oder ein salzbildendes Kation darstellt.

11. Präparat nach den Ansprüchen 1-8, worin die antibakteriell wirksame Verbindung Ceftriaxon oder ein pharmazeutisch anwendbares Salz, Ester oder Hydrat davon ist.

Europäisches Patentamt

# EUROPÄISCHER RECHERCHENBERICHT

Nummer der Anmeldung

EP 90 11 7309

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 126 348 (KYOTO PHARMACEUTICAL INDUSTRIES)<br>* Seiten 3,5-6 *<br>--- | 1-11 | A 61 K 31/545<br>A 61 K 31/43<br>A 61 K 31/71<br>A 61 K 9/02<br>A 61 K 47/14<br>A 61 K 47/12 |
| X | EP-A-0 152 896 (F. HOFFMANN-LA ROCHE & CO. AG)<br>* Seiten 12-14, Ansprüche 1-15 *<br>--- | 1-11 | |
| X | EP-A-0 108 295 (F. HOFFMANN-LA ROCHE & CO. AG)<br>* Seiten 29-31, Ansprüche 1-18 *<br>----- | 1-11 | |
| | | | RECHERCHIERTE SACHGEBIETE (Int. Cl.5) |
| | | | A 61 K |

Der vorliegende Recherchenbericht wurde für alle Patentansprüche erstellt

| Recherchenort | Abschlußdatum der Recherche | Prüfer |
|---|---|---|
| DEN HAAG | 17-12-1990 | BRINKMANN C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument